# EUROPÄISCHE PATENTSCHRIFT

(11) **EP 4 443 134 B1**
(45) Veröffentlichungstag und Bekanntmachung des Hinweises auf die Patenterteilung: **29.04.2026**
(21) Anmeldenummer: 24167294.8
(22) Anmeldetag: 28.03.2024
(51) Int. Cl.: G01N 17/04, G01N 33/00

(54) **VORRICHTUNG UND VERFAHREN ZUR ERKENNUNG EINES KORROSIVEN UMGEBUNGSKLIMAS IN EINEM SCHUTZGEHÄUSE**
APPARATUS AND METHOD FOR DETECTING A CORROSIVE ENVIRONMENTAL CLIMATE IN A CONTAINMENT ENCLOSURE
DISPOSITIF ET PROCÉDÉ DE DÉTECTION D'UN CLIMAT ENVIRONNEMENTAL CORROSIF DANS UN BOÎTIER DE PROTECTION

(30) Priorität: 03.04.2023 DE 102023108449
(43) Veröffentlichungstag der Anmeldung: 09.10.2024
(73) Patentinhaber: Turck Holding GmbH, 58553 Halver (DE)
(72) Erfinder: Ebinger, Klaus, 47906 Kempen (DE); Bauer-Wesely, Michael, 08344 Grünhain- Beierfeld (DE); Leonhardt, Christian, 09111 Chemnitz (DE); Schulze, Robert, 09117 Chemnitz (DE)
(74) Vertreter: DTS Patent- und Rechtsanwälte PartmbB

(56) Entgegenhaltungen:
- JP-A- 2012 189 356
- TW-B- I 787 971
- US-A1- 2015 268 152
- US-A1- 2018 149 579
- US-A1- 2020 333 272
- US-A1- 2022 057 279

## Beschreibung

Die vorliegende Erfindung betrifft eine Vorrichtung zur Erkennung eines korrosiven Umgebungsklimas in einem Schutzgehäuse, insbesondere Schaltschrank, und ein Verfahren zum Betreiben einer derartigen Vorrichtung.

Aus dem Stand der Technik ist es bekannt, Korrosion anhand von veränderten Materialeigenschaften eines Werkstücks, insbesondere mit Hilfe von optischen, elektrischen und/oder elektrochemischen Verfahren zu erkennen. Dies ist eine geeignete Vorgehensweise in Anwendungen, in denen Korrosion nicht vermeidbar ist und ein normales Verschleißverhalten beispielsweise eines Bauteils darstellt. Nachteilig an einer derartigen Vorgehensweise ist, dass die Korrosion an der durch den Messaufbau definierten Stelle bereits begonnen hat. Darüber hinaus gibt es über das Ausmaß der Korrosion an anderen Stellen, beispielsweise an anderen in einem Schutzgehäuse verbauten Komponenten, keine Erkenntnisse. Die bekannten Messverfahren sind teilweise sehr aufwendig und können oftmals nicht in Schaltschränken implementiert werden

Korrosion ist im technischen Umfeld insbesondere der Schalt- und Anlagentechnik ein Phänomen, welches sehr häufig unerwünschte Folgen nach sich zieht. Dazu zählen zum einen eine Veränderung von Materialeigenschaften der in einem Schaltschrank verbauten I/O- Komponenten oder - im Extremfall - die gänzliche Zerstörung einer dieser I/O- Komponenten. Insbesondere durch den aktuellen Trend der dezentralen Automation werden Schaltschränke und Schutzgehäuse bevorzugt maschinennah montiert und sind dadurch in einem erheblichen Maße gefährlichen und korrosiven Umgebungsbedingungen ausgesetzt.

Zur Korrosionserkennung ist beispielsweise aus der US 2022/0057279 A1 bekannt, eine korrosionsbedingte Beanspruchung innerhalb eines Schutzgehäuses mit Hilfe eines optischen Sensors, der Wellenlängenänderungen von im Schutzgehäuse reflektierter UV-Strahlung erfasst, zu bestimmen. Die US 2020/333272 A1 beschreibt eine Vorrichtung zur Vorhersage von Korrosion und Selbstentzündung in petrochemischen Anlagen. Aus der JP 2012-189356 A ist ein Verfahren bekannt, um die Lebensdauer von Leiterplatten in einer Umgebung abzuschätzen. TWI787971B offenbart eine Vorrichtung zur Vorhersage einer Korrosionsgeschwindigkeit, umfassend ein trainiertes Modell.

Zur Korrosionserkennung sind ferner aus dem Stand der Technik Sensoren mit Opferanoden aus unedlen Materialien bekannt, die typischerweise an exponierter Stelle angeordnet sind, um unter einem bestehenden korrosiven Umgebungsklima aktiv zu korrodieren. Ein derartiges Vorgehen ermöglicht wiederrum lediglich eine nachträgliche Korrosionserkennung, das heißt nach bereits erfolgter Korrosion, die beispielsweise anhand korrosionsbedingt veränderter elektrischer Eigenschaften, insbesondere der elektrischen Leitfähigkeit, der Opferanode festgestellt werden kann.

Im Bereich der Schutzgehäuse beziehungsweise Schaltschränke sind zudem aufwändige Klima-, Lüftungs- und/oder Filtersysteme bekannt, die dazu eingerichtet sind, gleichbleibende und möglichst nicht-korrosive Umgebungseinflüsse innerhalb des Schaltschranks einzustellen. Ein derartiges Vorgehen ist allerdings nur in einem Umfeld von sehr teuren Instrumenten wirtschaftlich sinnvoll, da die dazu notwendigen Komponenten, wie etwa gasdichtem Schaltschrank, Filteranlagen und so weiter, typischerweise technisch anspruchsvoll sind und insbesondere ständig gewartet und überwacht werden müssen.

Wünschenswert wäre es, den Grad einer beginnenden Korrosion frühzeitig zu erkennen oder vor deren Entstehung zu prognostizieren. Insbesondere in Schaltschränken und/oder ähnlich gearteten Schutzgehäusen kann unter Umständen bei korrosionsbedingtem Versagen der Schutzfunktion eine korrosive Atmosphäre eindringen, welches innerhalb kurzer Zeit zu einer Zerstörung der verbauten I/O - Komponenten führen kann.

In diesem Zusammenhang stellt sich die vorliegende Erfindung zur Aufgabe, eine Vorrichtung zur Erkennung eines korrosiven Umgebungsklimas in einem Schutzgehäuse, insbesondere Schaltschrank, und ein Verfahren zum Betrieb einer solchen Vorrichtung anzugeben, wobei eine Korrosion frühzeitig, insbesondere vor deren Entstehung, ermittelt werden kann.

Diese Aufgabe wird erfindungsgemäß gelöst durch eine Vorrichtung und ein Verfahren mit den Merkmalen der unabhängigen Ansprüche. Vorteilhafte Ausgestaltungen sind in den Unteransprüchen angegeben.

Danach wird die Aufgabe gelöst durch eine Vorrichtung zur Erkennung eines korrosiven Umgebungsklimas in einem Schutzgehäuse, insbesondere Schaltschrank, umfassend:
- zumindest zwei interne Sensoren, die dazu ausgebildet sind, die zeitlichen Verläufe von unterschiedlichen Umgebungsparametern, insbesondere einer Luft-und/oder einer Komponententemperatur, einer Luftfeuchte und/oder einer Gaskonzentration, innerhalb des Schutzgehäuses zu erfassen und ein den jeweiligen zeitlichen Verlauf des jeweiligen Umgebungsparameters widerspiegelndes Sensorsignal zu erzeugen;
- eine Auswerteinrichtung, die dazu eingerichtet ist, die die jeweiligen zeitlichen Verläufe der unterschiedlichen Umgebungsparameter widerspiegelnden Sensorsignale zu empfangen und in Korrelation zueinander auszuwerten, um eine Intensität des korrosiven Umgebungsklimas anhand von Abweichungen der Sensorsignale oder einer damit korrelierten Größe von einem Sollwert oder Sollwertbereich unter Berücksichtigung einer zeitlichen Dauer und/oder einer Häufigkeit der Abweichungen zu ermitteln; und
- eine Ausgabeeinrichtung, die dazu eingerichtet ist, ein die ermittelte Intensität des korrosiven Umgebungsklimas widerspiegelndes Ausgabesignal zu erzeugen.

Die zumindest zwei internen Sensoren sind insbesondre innerhalb des Schutzgehäuses, insbesondere in dem Schaltschrank, angeordnet.

Die Erfindung basiert unter anderem auf der Erfassung von relevanten Umgebungsparametern, wie etwa einer Luft- und/oder Komponententemperatur, einer relativen oder absoluten Luftfeuchtigkeit, einer Gaskonzentration, insbesondere von korrosiven Gasen, innerhalb des Schutzgehäuses beziehungsweise des Schaltschranks. Im Kern wird vorgeschlagen, nicht nur Grenzwerte, sondern darüber hinaus den zeitlichen Aspekt von sich verändernden Umgebungsbedingungen, welcher sich entsprechend in den zeitlichen Verläufen der erfassten Umgebungsparametern widerspiegelt, zu betrachten. Auf diese Weise wird bei der Auswertung der Sensorsignale insbesondere die Zeitspanne berücksichtigt, wie lange eine erfasste Kontamination dauert und wie häufig eine Kontamination auftritt, um aussagekräftige Informationen über das Auftreten einer korrosionsbegünstigen Atmosphäre zu erhalten und die Intensität des korrosiven Umgebungsklimas zuverlässig zu ermitteln.

Korrosionsbedingte Schäden, insbesondere an im Schutzgehäuse verbauten Komponenten, wie etwa I/O-Komponenten eines Schaltschrankes, können somit im Idealfall vermieden werden. Die Intensität des korrosiven Umgebungsklimas ist über die Ausgabeeinrichtung, beispielsweise einen Bildschirm, eine LED-Anzeige oder Ähnliches, ausgebbar, so dass insbesondere dem Anwender eine Warnung, beispielsweise in Form von Warnsignalen einer Ampel, ausgebbar ist, wenn ein korrosives Umgebungsklima mit erhöhter Intensität festgestellt wurde. Die Ausgabe kann ferner über eine datentechnische Anbindung an ein Netzwerk oder einen Cloud-Dienst erfolgen, es kann eine elektronische Nachricht, E-Mail oder Kurznachricht (SMS) verschickt werden oder es kann eine Warnmeldung bei einer übergeordneten Steuerungsebene ausgegeben werden.

In anderen Anwendungsbeispielen ist eine Handlungsempfehlung in Abhängigkeit der ermittelten Intensität der korrosiven Umgebungsklimas ausgebbar. Alternativ oder zusätzlich sind Aktoren zum Ausführen von entsprechender Sofortmaßnahmen nach Maßgabe der Ausgangssignals ansteuerbar, sodass eine von der ermittelten Intensität des korrosiven Umgebungsklimas abhängige Steuerung implementiert ist. Beispielsweise ist gemäß unterschiedlichen Ausgestaltungen eine Heizung, eine Kühlung, eine Klimaanlage, eine Absaugvorrichtung und/oder eine Filteranlage mit der Auswerteinrichtung derart verbunden, dass diese nach Maßgabe des Ausgangssignals ansteuerbar ist und insbesondere in Abhängigkeit von der ermittelten Intensität des korrosiven Umgebungsklimas aktiviert werden kann.

Insbesondere im Bereich der Anlagentechnik zieht eine nicht erkannte Korrosion oftmals eine Einschränkung der Funktion beziehungsweise - im Extremfall - einen unplanmäßigen Totalausfall mit nachgängigem Stopp einer kompletten Anlage oder Maschine nach sich. Durch Korrosion verursachte Schäden können auch negative Einflüsse auf Sicherheitsparameter (FUSI, Ex) einer Anlage haben, insbesondere bei Schaltschränken. Es ist daher vorteilhaft, das Risiko einer Korrosionsentstehung frühzeitig zu erkennen, um gegebenenfalls Gegenmaßnahmen einläuten zu können.

Die Berechnung beziehungsweise Ermittlung der Intensität des korrosiven Umgebungsklimas kann in Ausgestaltungen in einer lokalen Auswerteinrichtung, insbesondere Prozessor oder Mikrochip, oder mit Hilfe einer Cloudanwendung, die beispielsweise über ein Intranet oder dem Internet angebunden ist, erfolgen. Die Übertragung der Signale, insbesondere Sensorsignale und/oder Ausgangssignale, erfolgt in Ausgestaltungen kabelgebunden oder drahtlos, beispielsweise via Lichtwellenleiter, WLAN oder dergleichen.

Die Vorrichtung ist in möglichen Ausführungsbeispielen insbesondere als Nachrüstsatz zur Installation in bestehende Schaltschränke ausgebildet.

Die Auswerteinrichtung umfasst ferner ein trainiertes Modell, insbesondere Deep-Learning Modell, welches in einer Anlernphase dazu trainiert wurde, die Intensität des korrosiven Umgebungsklimas anhand der zeitlichen Verläufe der unterschiedlichen Umgebungsparameter zu klassifizieren. Der Einsatz von künstlicher Intelligenz zur Ermittlung der Intensität des korrosiven Umgebungsklimas ist besonders vorteilhaft, da es nicht notwendig ist, diese als Funktion von Material-, Umgebungs- und/oder weiteren Umfeldparametern zu spezifizieren. Vielmehr kann das Modell mit Hilfe von empirisch bestimmten Anlerndaten dazu trainiert werden, die Intensität des korrosiven Umgebungsklimas anhand der Sensorsignale zu klassifizieren.

Beispielsweise erfolgt das, insbesondere überwachte, Anlernen beziehungsweise Trainieren des Modells im Betrieb von Test-Aufbauten, die unter gegebenen Umgebungseinflüssen, welche mittels der internen Sensoren ständig überwacht und erfasst werden. Diese Aufbauten werden bis zu beginnender Korrosion vorzugsweise nicht gewartet, um Anlerndaten zu erhalten, die zum Trainieren des Modells geeignet sind.

Bei einer weiteren Ausbildung ist die Auswerteinrichtung dazu ausgebildet, die Intensität des korrosiven Umgebungsklimas unter Berücksichtigung eines vorab, insbesondere in einer Initialisierungsphase und/oder in der vorstehend bereits genannten Anlernphase, empirisch ermittelten Korrosionsverhaltens eines korrodierenden Probekörpers zu ermitteln. Der Probekörper besteht typischerweise aus einem unedlen Material, dessen Korrosivität im Vergleich zu anderen im Schutzgehäuse verbauten Materialien quantitativ bekannt ist und/oder ermittelt werden kann.

Um eine Veränderung von korrosiven Umgebungsbedingungen feststellen zu können, ist es unter Umständen zielführend, das Maß der Korrosivität bei der Initialisierung zu erfassen. Dies erfolgt beispielsweise über den vorstehend genannten Probekörper. Der Probekörper besteht aus einem unedlen Material, welches seine elektrischen Eigenschaften, insbesondere seine elektrische Leitfähigkeit, über einen definierten Zeitraum der Initialisierung verändert. In diesem definierten Zeitraum wird die zeitliche Veränderung einer elektrischen Eigenschaft, insbesondere elektrischen Leitfähigkeit, erfasst. Der Probekörper wird an einer exponierten Stelle im Schutzgehäuse beziehungsweise Schaltschrank montiert. Nach der Initialerfassung beziehungsweise Initialisierung wird der Probekörper nicht mehr benötigt und kann somit auch entfernt werden. Bei veränderten Umgebungsbedingungen kann die Initialisierung jedoch jederzeit wiederholt werden.

Anhand der während der Initialisierung gewonnenen Daten kann eine etwaige korrosive Umgebung als Ausgangszustand ermittelt werden. Im Betrieb erfolgt eine weitere Erfassung der oben genannten Umgebungsparameter, wie etwa Temperatur, Feuchtigkeit und/oder Gaskonzentration von korrosiven Gasen. Die dabei erhaltenen Werte und zeitlichen Verläufe der Umgebungsparametern können mit denjenigen des Ausgangszustands verglichen werden, um daraus Handlungsmaßnahmen an den Anwender abzuleiten und auszugeben. Insbesondere kann so festgestellt werden, ob hinsichtlich der Korrosion gleichbleibende, verbesserte und/oder verschlechterte Umgebungsbedingungen vorliegen.

Die Initialisierungsphase dient in möglichen Ausgestaltungen der Erfindung zum Anlernen des der Auswertung zugrunde liegenden Modells, insbesondere Deep-Learning Modells; insofern ist vorgesehen, Daten, die bei der vorstehend beschriebenen Initialisierung insbesondere bezüglich korrosionsbedingt veränderter Materialeigenschaften des Probekörpers anfallen, als Anlerndaten für das zu trainierende Modell zu verwenden. Die Anlerndaten des Modells beinhalten somit in möglichen Ausgestaltungen der Erfindung insbesondere Informationen über die zeitliche Dauer und Häufigkeit von Abweichungen der Umgebungsparametern von einem vorgegebenen oder vorgebbaren Sollwert oder Sollwertbereich während der Initialisierung. Die der Ermittlung der Intensität des korrosiven Umgebungsklimas zu Grunde liegende Korrelationen mit den Sensorsignalen ist bei derartig trainierten Modellen somit vorgegeben beziehungsweise angelernt.

Bei einer weiteren Ausbildung ist zumindest einer der internen Sensoren als Luftfeuchtesensor und zumindest ein weiterer der internen Sensoren als Temperatursensor eingerichtet. Die Auswerteinrichtung ist dazu ausgebildet, die Intensität des korrosiven Umgebungsklimas unter Berücksichtigung eines anhand der Sensorsignale des Luftfeuchtesensors und des Temperatursensors berechneten Taupunktes zu ermitteln.

Mit anderen Worten wird der Taupunkt als zumindest eine der Größen, die mit den erfassten Umgebungsparametern korreliert ist, bestimmt und bei der Ermittlung der Intensität des korrosiven Umgebungsklimas zugrunde gelegt. Insbesondere im Fall, dass sich die Temperatur an einer Komponente, insbesondere I/O - Komponente, oder an einer Gehäuse- oder Schaltschrankwand dem Taupunkt annähert oder diesen unterschreitet, ist mit korrosionsbegünstigender Kondensation zu rechnen.

Vorzugsweise sind die internen Sensoren an definierten Stellen im Schutzgehäuse beziehungsweise Schaltschrank verbaut, um eine umfängliche Darstellung der korrosiven Umgebungsbedingungen zu ermöglichen. Besonders bevorzugt sind die internen Sensoren derart angeordnet, dass Extremstellen der Temperaturverteilung innerhalb des Schutzgehäuses beziehungsweise Schaltschranks, wie etwa Coldspots oder Hotspots, erfassbar sind. Auf diese Weise kann insbesondere eine im Schutzgehäuses beziehungsweise Schaltschranks beginnende Kondensation frühzeitig erkannt werden.

In Ausgestaltungen mit mehreren Temperatursensoren sind diese bevorzugt so angeordnet, dass ein großer gemessener Temperaturunterschied zwischen den entsprechenden Messpunkten zu erwarten ist.

Bei einer weiteren Ausbildung ist der Temperatursensor dazu eingerichtet, die Temperatur einer Kaltstelle innerhalb des Schutzgehäuses zu erfassen, wobei der Temperatursensor an der Kaltstelle einen im Wesentlichen isolierten Messpunkt definiert oder als Flächenfühler ausgebildet ist. Insbesondere bei Schaltschränken sind Kaltstellen oftmals im Bereich einer Außenwand und/oder in der Nähe einer etwaig vorhandenen Klimatisierung und/oder Kühlung zu finden, sodass es sich anbietet, die dort lokal vorherrschenden Temperaturen zu erfassen, um eine korrosionsbegünstigende Kondensation frühzeitig erkennen zu können.

Bei einer weiteren Ausbildung ist der Temperatursensor als optischer Sensor, insbesondere als IR-Sensor, ausgebildet. In möglichen Anwendungen ist beispielsweise ein IR-Sensor auf den kältesten Punkt einer Schrankaußenwand oder auf einen Bereich einer Lüftung oder Kühlung eines Schaltschranks gerichtet, um von der Temperatur abhängige Sensorsignale bereitzustellen.

Bei einer weiteren Ausbildung ist zumindest einer der internen Sensoren der Vorrichtung zur Erkennung des korrosiven Umgebungsklimas zur Erfassung einer Gaskonzentration eines korrosiven Gases, insbesondere einer Halogenid-IonenKonzentration, einer Chlorid- und/oder einer Schwefelwasserstoffkonzentration, innerhalb des Schutzgehäuses eingerichtet. Die Existenz von korrosiven, halogenhaltige Verbindungen enthaltenden Gasen, insbesondere mit in Wasser gelöstem Chlorid, kann insbesondere durch einen Standort in Meeresnähe verursacht sein. Schwefelwasserstoffhaltige Gase können beispielsweise bei Standorten in der Nähe von chemischen Anlagen auftreten.

In möglichen Ausbildungen ist einer der internen Sensoren als Türsensor ausgebildet und dazu eingerichtet als Umgebungsparameter zu erfassen, ob eine Tür des Schutzgehäuses geöffnet oder verschlossen ist. Eine geöffnete Tür begünstigt im Allgemeinen die Korrosionsentstehung.

Bei einer weiteren Ausbildung ist die Auswerteinrichtung dazu ausgebildet, lokale Klimadaten und/oder geographische Standortdaten, welche vom geographischen Standort des Schutzgehäuses abhängige Informationen beinhalten, zu empfangen und bei der Ermittlung der Intensität des korrosiven Umgebungsklimas zu berücksichtigen. Hierzu kann die Auswerteinrichtung beispielsweise dazu ausgebildet sein, Wetterdaten einer lokalen Wetterstation oder entsprechender externer Sensoren zu empfangen.

Die geographische Lage der Installation kann zuverlässig beispielsweise via GPS (Global Positioning System) oder einem ähnlichen Satelliten basierten Navigationssystem erhalten werden. Insbesondere in Verbindung mit Wetterdaten betreffend Außentemperatur, Windgeschwindigkeit und so weiter können bei der Ermittlung der Intensität des korrosiven Umgebungsklimas lokale Korrelationen mit standortabhängigen, externen Umwelteinflüssen berücksichtigt werden. Beispielsweise kann eine in Meeresnähe installierte Vorrichtung in Abhängigkeit von der Windrichtung und/oder -geschwindigkeit einer erhöhten Salzbeziehungsweise Chlorid-Konzentration ausgesetzt sein.

Die geografischen Standortdaten spezifizieren bevorzugt neben der geografischen Lage beispielsweise auch den Expositionsgrad gegenüber den äußeren klimatischen Bedingungen, wie etwa ob es sich um eine Installation in einer Halle oder im Freien handelt. Bei Installationen innerhalb von Anlagen kann beispielsweise ein erhöhtes Kondensationsrisiko aufgrund von Aggregationskeimen, wie etwa Staub, bestehen.

Anhand der geografischen Standortdaten können insbesondere auch betriebsbedingte Tag- / Nacht-Intervalle, je nach Jahreszeit, bei der Ermittlung der Intensität des korrosiven Umgebungsklimas berücksichtigt werden.

Die vorstehend und nachfolgenden beschriebene Vorteile der hier offenbarten Vorrichtung betreffen insbesondere Schaltschränke der Elektrotechnik, beispielsweise der industriellen Anlagetechnik, in welchen I/O- Komponenten, wie etwa Messumformer oder anderweitige elektronische Schaltkomponenten, angeordnet sind, die mit Feldgeräten, Sensoren und Aktoren einer industriellen Anlage kommunizieren. Die Erfindung betrifft gleichermaßen ein Schutzgehäuse, insbesondere einen Schaltschrank, mit der hier beschriebenen Vorrichtung zur Erkennung des korrosiven Umgebungsklimas.

Bei einem Verfahren zum Betreiben der vorstehend beschriebenen Vorrichtung werden die zeitlichen Verläufe von zumindest zwei unterschiedlichen Umgebungsparametern, insbesondere der Luft- und/oder der Komponententemperatur, der Luftfeuchte und/oder der Gaskonzentration, mittels innerhalb des Schutzgehäuses angeordneter, interner Sensoren erfasst, wobei ein den jeweiligen zeitlichen Verlauf des jeweiligen Umgebungsparameters widerspiegelndes Sensorsignal erzeugt wird. Die die jeweiligen zeitlichen Verläufe der Umgebungsparameter widerspiegelnden Sensorsignale werden zur Auswertung in Korrelation zueinander gebracht. Die Intensität des korrosiven Umgebungsklimas wird anhand von Abweichungen der Sensorsignale oder einer damit korrelierten Größe von einem Sollwert oder Sollwertbereich unter Berücksichtigung einer zeitlichen Dauer und einer Häufigkeit der Abweichungen ermittelt. Entsprechend diesem Ergebnis wird ein Ausgabesignal, welches die ermittelte Intensität des korrosiven Umgebungsklimas widerspiegelt, erzeugt.

Vorteile und Wirkungsweise des erfindungsgemäßen Verfahrens ergeben sich unmittelbar aus der bisherigen und nachfolgenden Beschreibung mit Bezug auf die Vorrichtung zur Erkennung eines korrosiven Umgebungsklimas.

Bei der Auswertung der Sensorsignale wird insbesondere die Zeitspanne berücksichtigt, wie oft und lange die Sensorsignale von einem Sollwert oder Sollwertbereich abweichen. Mit anderen Worten wird die Dauer und Häufigkeit von Korrosion begünstigenden Kontaminationen berücksichtigt, um mögliche korrosionsbedingte Schäden prognostizieren zu können.

In Abhängigkeit der Intensität des korrosiven Umgebungsklimas werden mittels des Ausgabesignals insbesondere Handlungsanweisungen an einen Anwender ausgegeben oder entsprechende Aktoren zur Ausführung von geeigneten Maßnahmen automatisch angesteuert. Beispielsweise wird eine Klimaanlage, Lüftung oder Heizung aktiviert, insbesondere wenn der Taupunkt mehrmals oder nachhaltig unterschritten wurde.

Bei einer weiteren verfahrenstechnischen Ausgestaltung der Erfindung wird in einer Anlernphase und/oder Initialisierungsphase zumindest ein Probekörper aus einem Probematerial innerhalb des Schutzgehäuses angeordnet und zumindest eine elektrische Eigenschaft des Probekörpers über einen vorgegebenen Zeitraum unter gegebenen Umgebungseinflüssen erfasst, wobei das Probematerial in Korrelation gesetzt wird zu anderen im Schutzgehäuse verbauten Materialien und bei der Ermittlung der Intensität des korrosiven Umgebungsklimas berücksichtigt wird.

Bei einer möglichen verfahrenstechnischen Ausbildung wird
- eine empirische Datenbasis von typischerweise in einem Schaltschrank verbauten Metallen erstellt;
- eine Korrelation von Materialeigenschaften dieser Metalle zu denjenigen des Probekörpers bestimmt; und
- der Einfluss der vorliegenden Umgebungsbedingungen als Funktion der Zeit in Abhängigkeit von Temperatur und relativer Luftfeuchte sowie der Gaskonzentration in Abhängigkeit des Standortes (z. B. Salzgehalt in der Luft beziehungsweise Nähe zum Meer) bestimmt und zur Prognose von korrosionsbedingten Ausfällen vor deren Entstehung berücksichtigt. Eine Beziehung zwischen den Materialeigenschaften des Probekörpers und den übrigen im Schaltschrank verbauten Metallen kann beispielsweise über das elektrochemische Potential beziehungsweise auf Basis der Nernst-Gleichung etabliert werden.

Weitere Einzelheiten und Vorteile der Erfindung sollen nun anhand der in den Zeichnungen dargestellten Ausführungsbeispiele näher erläutert werden.

Es zeigen:
- Fig. 1: eine schematische Darstellung eines möglichen Ausführungsbeispiels der Vorrichtung;
- Fig. 2: ein Blockdiagramm, welches ein Verfahren zum Betrieb der Vorrichtung der Fig. 1 illustriert.

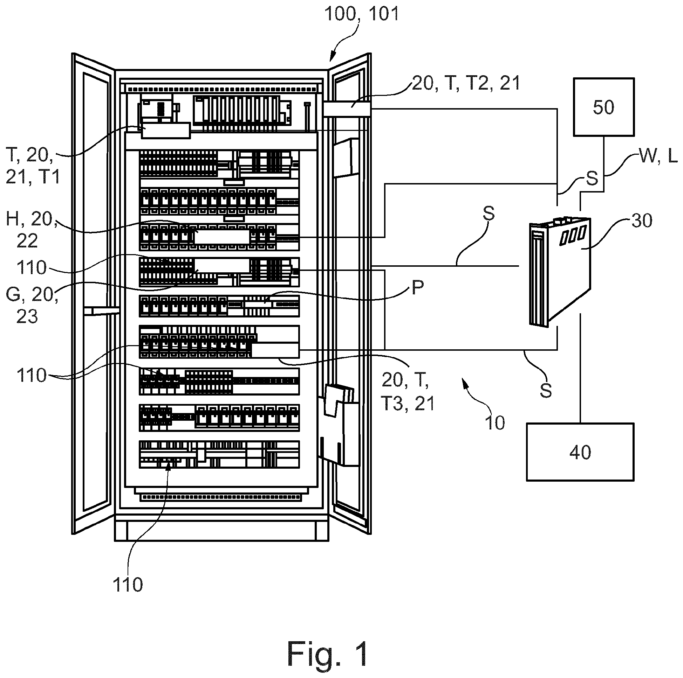

Mit Bezug zu Fig. 1 wird ein erstes Ausführungsbeispiel der Vorrichtung 10 zur Erkennung eines korrosiven Umgebungsklimas in einem Schutzgehäuse 100, bei dem es sich in dem dargestellten Beispiel um einen Schaltschrank 101 handelt, erläutert. Im Schaltschrank 101 sind eine Vielzahl von I/O-Komponenten 110 angeordnet, die mit Feldgeräten, Sensoren und Aktoren einer industriellen Anlage schalttechnisch verbunden sind.

Die Vorrichtung 10 umfasst mehrere interne Sensoren 20, die dazu ausgebildet sind, unterschiedliche Umgebungsparameter T, H, G kontinuierlich oder in wiederkehrenden Zeitintervallen zu erfassen und jeweils zugeordnete Sensorsignale S zu erzeugen, die einen zeitlichen Verlauf des jeweiligen erfassten Umgebungsparameters T, H, G repräsentieren.

Die internen Sensoren 20 sind mit einer Auswerteinrichtung 30 zur Auswertung der Sensorsignale S kabelgebunden oder drahtlos verbunden.

Die Sensorsignale S sind beispielsweise digitale oder analoge Signale.

Bei der Auswerteinrichtung 30 kann es sich beispielsweise um eine lokale Auswerteinrichtung, wobei die Auswerteinrichtung 30 insbesondere innerhalb des Schutzgehäuses 100 angeordnet sein kann, oder um eine Auswerteinrichtung einer Datencloud in einem Intranet oder dem Internet handeln.

Im dargestellten Ausführungsbeispiel sind drei der internen Sensoren 20 als Temperatursensoren 21 ausgebildet und zur Erfassung von Temperaturen T in unterschiedlichen Bereichen des Schaltschranks 101 angeordnet. Dabei ist einer der Temperatursensoren 21 zur Messung einer Lufttemperatur T1 eingerichtet, ein weiterer Temperatursensor 21 befindet sich zur Temperaturmessung T2 einer Kaltstelle im Bereich einer Schaltschrankwand oder Tür. Ein Temperatursensor 20 ist zur Messung einer Komponententemperatur T3 im Bereich einer I/O-Komponente 110 des Schaltschranks 101 angeordnet.

Die Temperatursensoren 21 sind vorzugsweise als optische Sensoren, insbesondere als IR-Sensoren, ausgebildet.

Die Vorrichtung 10 umfasst ferner einen internen Sensor 20, der als Luftfeuchtesensor 22 zur Erfassung einer Luftfeuchte H innerhalb des Schutzgehäuses 100 beziehungsweise Schaltschranks 101 eingerichtet ist. Ein zusätzlicher interner Sensor 20 ist als Gassensor 23 zur Erfassung einer Konzentration G von korrosionsbegünstigenden Stoffen, wie zum Beispiel eines korrosiven Gases, insbesondere einer halogenhaltigen Verbindung, eines Chlorid oder Schwefelwasserstoff enthaltenden Gases oder eines entsprechenden in Wasser gelösten Stoffes, Verschmutzungen durch Feststoffe oder auch in Flüssigkeiten gelöster Stoffe ausgebildet.

Die erzeugten Sensorsignale S repräsentieren jeweils den zeitlichen Verlauf der erfassten Umgebungsparameter T, H, G. Die Auswerteinrichtung 30 ist dazu eingerichtet, die den Umgebungsparametern T, H, G zugeordneten Sensorsignale S zu empfangen und in Korrelation zueinander zu bringen, um hieraus eine Intensität des korrosiven Umgebungsklimas abzuleiten. Konkret wird die Intensität des korrosiven Umgebungsklimas anhand von Abweichungen der Sensorsignale S oder einer damit korrelierten Größe von einem Sollwert oder Sollwertbereich unter Berücksichtigung einer zeitlichen Dauer und einer Häufigkeit der Abweichungen bestimmt. Eine Ausgabeeinrichtung 40 ist dazu eingerichtet, ein Ausgabesignal zu erzeugen, welches die ermittelte Intensität des korrosiven Umgebungsklimas widerspiegelt.

Die Ausgabeeinrichtung 40 ist beispielsweise dazu eingerichtet, ein Warnsignal auszugeben, wenn die Intensität des korrosiven Umgebungsklimas einen kritischen Wert erreicht. Im Allgemeinen kann das Ausgabesignal dazu dienen, einem Anwender Handlungsanweisungen entsprechend der ermittelten Intensität des korrosiven Umgebungsklimas zu signalisieren.

In anderen Ausführungsbeispielen werden mit Hilfe des Ausgangssignals Aktoren zur Durchführung von Sofortmaßnahmen angesteuert. Hierbei ist beispielsweise vorgesehen, eine Heizung, eine Kühlung, eine Klimaanlage und/oder eine Filteranlage in Abhängigkeit der ermittelten Intensität des korrosiven Umgebungsklimas automatisch anzusteuern.

Zur Auswertung berechnet die Auswerteinrichtung 30 als korrelierte Größe beispielsweise einen Taupunkt aus den vom Luftfeuchtesensor 22 und von den Temperatursensoren 21 bereitgestellten Sensorsignalen S beziehungsweise Daten. Abweichungen des berechneten Taupunkts von einem Sollwert oder Sollwertbereich deuten auf eine korrosionsbegünstigende Kondensation innerhalb des Schaltschranks 101 hin. Die Intensität des korrosiven Umgebungsklimas wird in Abhängigkeit der zeitlichen Dauer und Häufigkeit dieser Abweichungen ermittelt.

Zur Ermittlung der Intensität des korrosiven Umgebungsklimas umfasst die Auswerteinrichtung 30 beispielsweise ein trainiertes Modell, insbesondere Deep-Learning Modell, welches in einer Initialisierungs- beziehungsweise Anlernphase dazu trainiert wurde, die Intensität des korrosiven Umgebungsklimas anhand der zeitlichen Verläufe der unterschiedlichen Umgebungsparameter T, H, G zu klassifizieren. Beispielsweise ist die Auswerteinrichtung 30 dazu ausgebildet, die Intensität des korrosiven Umgebungsklimas unter Berücksichtigung eines vorab, insbesondere in einer Initialisierungsphase und/oder in der Anlernphase, empirisch ermittelten Korrosionsverhaltens eines korrodierenden Probekörpers P zu ermitteln.

Der Probekörper P besteht beispielsweise aus einem unedlen Probematerial, insbesondere Metall, dessen elektrische Eigenschaften, insbesondere Leitfähigkeit, während des Anlernens beziehungsweise während der Initialisierung unter dem Einfluss von Korrosion empirisch bestimmt werden. Dazu wird der Probekörper P typischerweise an einer exponierten Stelle im Schutzgehäuse 100 beziehungsweise Schaltschrank 101 montiert. Das Verhalten des Probekörpers P unter dem Einfluss von Korrosion lässt sich beispielsweise unter Berücksichtigung des elektrochemischen Potentials beziehungsweise der Nernst-Gleichung, zu anderen im Schutzgehäuse 100 verbauten Metallen beziehungsweise Materialien in Beziehung setzen.

Die Auswerteinrichtung 30 ist ferner dazu ausgebildet, lokale Klimadaten W und/oder lokale geographische Standortdaten L, welche Informationen über das lokale Wetter und/oder den geographischen Standort des Schutzgehäuses 100 beinhalten, zu empfangen und bei der Ermittlung der Intensität des korrosiven Umgebungsklimas zu berücksichtigen. Die Auswerteinrichtung 30 steht hierzu beispielsweise in einer Datenverbindung mit einer Wetterstation oder entsprechend ausgebildeten, externen Sensoren 50. Die Wetterdaten beinhalten vorzugsweise Informationen über eine Außentemperatur, Windgeschwindigkeit, Niederschlagswahrscheinlichkeit, und so weiter, die einen Einfluss auf das im Schutzgehäuse 100 vorherrschende, korrosive Umgebungsklima haben können. Beispielsweise kann die Vorrichtung 10 in Abhängigkeit der Windrichtung und ihrem geographischen Standort, insbesondere mit Bezug auf Salzwasserkörper oder Anlagenkomponenten einer chemischen Anlage, einer erhöhten Exposition durch korrosive Gase ausgesetzt sein.

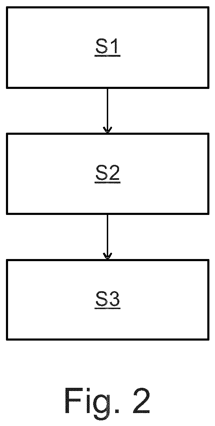

Ein Verfahren zum Betreiben der vorstehend beschriebenen Vorrichtung 10 ist in dem Blockdiagramm der Fig. 2 schematisch illustriert. Bei dem Verfahren zum Betreiben der vorstehend beschriebenen Vorrichtung 10 werden in einem ersten Schritt S1 die zeitlichen Verläufe der unterschiedlichen Umgebungsparametern T, H, G, insbesondere der Luft- und/oder der Komponententemperatur, der Luftfeuchte und/oder der Gaskonzentration, mit Hilfe der internen Sensoren 20 erfasst und ein den jeweiligen zeitlichen Verlauf des jeweiligen Umgebungsparameters T, H, G widerspiegelndes Sensorsignal S erzeugt. Die die jeweiligen zeitlichen Verläufe der Umgebungsparameter T, H, G widerspiegelnde Sensorsignale S werden in einem zweiten Schritt S2 zur Auswertung in Korrelation zueinander gebracht, wobei die Intensität des korrosiven Umgebungsklimas anhand von Abweichungen von einem Sollwert oder Sollwertbereich unter Berücksichtigung der zeitlichen Dauer und der Häufigkeit der Abweichungen ermittelt. In einem dritten Schritt wird ein Ausgabesignal, welches die ermittelte Intensität des korrosiven Umgebungsklimas widerspiegelt, erzeugt.

Zum Anlernen beziehungsweise Initialisieren der Vorrichtung 10 wird der Probekörper P in einer Anlernphase beziehungsweise Initialisierungsphase innerhalb des Schutzgehäuses 100 angeordnet. Während der Anlernphase beziehungsweise Initialisierungsphase wird eine elektrische Eigenschaft des Probekörpers, insbesondere dessen elektrische Leitfähigkeit, über einen vorgegebenen Zeitraum unter gegebenen korrosiven Umgebungseinflüssen erfasst, um den Einfluss der Korrosion auf das Probematerial zu ermitteln. Dieses empirisch bestimmte Korrosionsverhalten des Probekörpers P wird in Korrelation gesetzt zu anderen im Schutzgehäuse verbauten Materialien und bei der Ermittlung der Intensität des korrosiven Umgebungsklimas entsprechend berücksichtigt. In Weiterbildung ist vorgesehen, eine empirische Datenbank von typischerweise in einem Schaltschrank verbauten Metallen zu erstellen und Korrelationen von Materialeigenschaften dieser Metalle zu denjenigen des Probekörpers anhand des elektrochemischen Potentials beziehungsweise auf Grundlage der Nernst-Gleichung zu bestimmen. Der Einfluss der während des Betriebs vorliegenden Umgebungsbedingungen wird sensorisch als Funktion der Zeit in Abhängigkeit der erfassten Temperatur T, Luftfeuchte H und/oder Gaskonzentration G bestimmt und bei der Prognose des korrosiven Umgebungsklimas berücksichtigt.

Zusammenfassend ist es ein grundlegendes Konzept der Erfindung, nicht nur Grenzwerte, sondern darüber hinaus den zeitlichen Aspekt von sich verändernden Umgebungsbedingungen zur Ermittlung der Intensität des korrosiven Umgebungsklimas heranzuziehen. Die Auswertung der Sensorsignale berücksichtigt die Dauer und Häufigkeit von erfassten Kontaminationen, um aussagekräftige Informationen über das Auftreten einer korrosionsbegünstigen Atmosphäre zu erhalten und die Intensität des korrosiven Umgebungsklimas zuverlässig zu ermitteln.

### Bezugszeichenliste

- 10: Vorrichtung
- 20: interner Sensor
- 21: Temperatursensor
- 22: Luftfeuchtesensor
- 23: Gassensor
- 30: Auswerteinrichtung
- 40: Ausgabeeinrichtung

- 100: Schutzgehäuse
- 101: Schaltschrank
- 110: I/O-Komponente

- T: Umgebungsparameter, Temperatur
- H: Umgebungsparameter, Luftfeuchte
- G: Umgebungsparameter, Gaskonzentration
- S: Sensorsignal
- T1: Temperatur
- T2: Temperatur
- T3: Temperatur
- P: Probekörper
- W: lokale Klimadaten
- L: geographischen Standort

## Patentansprüche

1. Vorrichtung (10) zur Erkennung eines korrosiven Umgebungsklimas in einem Schutzgehäuse (100), insbesondere Schaltschrank (101), umfassend
- zumindest zwei interne Sensoren (20), die dazu ausgebildet sind, die zeitlichen Verläufe von unterschiedlichen Umgebungsparametern (T, H, G), insbesondere einer Luft- und/oder einer Komponententemperatur (T1, T2, T3), einer Luftfeuchte (H) und/oder einer Gaskonzentration (G), innerhalb des Schutzgehäuses (100) zu erfassen und ein den jeweiligen zeitlichen Verlauf des jeweiligen Umgebungsparameters (T, H, G) widerspiegelndes Sensorsignal (S) zu erzeugen;
- eine Auswerteinrichtung (30), die dazu eingerichtet ist, die die jeweiligen zeitlichen Verläufe der unterschiedlichen Umgebungsparameter (T, H, G) widerspiegelnden Sensorsignale (S) zu empfangen und in Korrelation zueinander auszuwerten, um eine Intensität des korrosiven Umgebungsklimas anhand von Abweichungen der Sensorsignale (S) oder einer damit korrelierten Größe von einem Sollwert oder Sollwertbereich unter Berücksichtigung einer zeitlichen Dauer und/oder einer Häufigkeit der Abweichungen zu ermitteln; und
- eine Ausgabeeinrichtung (40), die dazu eingerichtet ist, ein die ermittelte Intensität des korrosiven Umgebungsklimas widerspiegelndes Ausgabesignal zu erzeugen;
**dadurch gekennzeichnet, dass**
die Auswerteinrichtung (30) ein trainiertes Modell, insbesondere Deep-Learning Modell, umfasst, welches in einer Anlernphase dazu trainiert wurde, die Intensität des korrosiven Umgebungsklimas anhand der zeitlichen Verläufe der unterschiedlichen Umgebungsparameter (T, H, G) zu klassifizieren.

2. Vorrichtung (10) gemäß einem der vorhergehenden Ansprüche,
**dadurch gekennzeichnet, dass**
die Auswerteinrichtung (30) dazu ausgebildet ist, die Intensität des korrosiven Umgebungsklimas unter Berücksichtigung eines vorab, insbesondere in einer Initialisierungsphase und/oder in der Anlernphase, empirisch ermittelten Korrosionsverhaltens eines korrodierenden Probekörpers (P) zu ermitteln.

3. Vorrichtung (10) gemäß einem der vorhergehenden Ansprüche,
**dadurch gekennzeichnet, dass**
zumindest einer der internen Sensoren (20) als Luftfeuchtesensor (22) und zumindest weiterer der internen Sensoren (20) als Temperatursensor (21) eingerichtet ist und die Auswerteinrichtung (30) dazu ausgebildet ist, die Intensität des korrosiven Umgebungsklimas unter Berücksichtigung eines anhand der Sensorsignale (S) des Luftfeuchtesensors (22) und des Temperatursensors (22) berechneten Taupunktes zu ermitteln.

4. Vorrichtung (10) gemäß Anspruch 3,
**dadurch gekennzeichnet, dass**
der Temperatursensor (21) dazu eingerichtet ist, die Temperatur (T3) einer Kaltstelle innerhalb des Schutzgehäuses (100) zu erfassen, wobei der Temperatursensor (21) an der Kaltstelle einen im Wesentlichen isolierten Messpunkt definiert oder als Flächenfühler ausgebildet ist.

5. Vorrichtung (10) gemäß Anspruch 3 oder 4,
**dadurch gekennzeichnet, dass**
der Temperatursensor (21) als optischer Sensor, insbesondere als IR-Sensor, ausgebildet ist.

6. Vorrichtung (10) gemäß einem der vorhergehenden Ansprüche,
**dadurch gekennzeichnet, dass**
zumindest einer der internen Sensoren (20) zur Erfassung einer Gaskonzentration (G) eines korrosiven Gases, insbesondere einer Chlorid- und/oder einer Schwefelwasserstoffkonzentration, innerhalb des Schutzgehäuses (100) eingerichtet ist.

7. Vorrichtung (10) gemäß einem der vorhergehenden Ansprüche,
**dadurch gekennzeichnet, dass**
die Auswerteinrichtung (30) dazu ausgebildet ist, lokale Klimadaten (W) und/oder geographische Standortdaten (L), welche vom geographischen Standort des Schutzgehäuses (100) abhängige Informationen beinhalten, zu empfangen und bei der Ermittlung der Intensität des korrosiven Umgebungsklimas zu berücksichtigen.

8. Verfahren zum Betreiben einer Vorrichtung (10) gemäß einem der vorhergehenden Ansprüche,
**dadurch gekennzeichnet, dass**
- die zeitlichen Verläufe von zumindest zwei unterschiedlichen Umgebungsparametern (T, H, G), insbesondere der Luft- und/oder der Komponententemperatur (T1, T2, T3), der Luftfeuchte (H) und/oder der Gaskonzentration (G), mittels innerhalb des Schutzgehäuses angeordneter, interner Sensoren (20) erfasst werden und ein den jeweiligen zeitlichen Verlauf des jeweiligen Umgebungsparameters (T, H, G) widerspiegelndes Sensorsignal (S) erzeugt wird;
- die die jeweiligen zeitlichen Verläufe der unterschiedlichen Umgebungsparameter (T, H, G) widerspiegelnden Sensorsignale (S) zur Auswertung in Korrelation zueinander gebracht werden; und
- die Intensität des korrosiven Umgebungsklimas anhand von Abweichungen der Sensorsignale (S) oder einer damit korrelierten Größe von einem Sollwert oder Sollwertbereich unter Berücksichtigung einer zeitlichen Dauer und einer Häufigkeit der Abweichungen ermittelt wird; und
- ein die ermittelte Intensität des korrosiven Umgebungsklimas widerspiegelndes Ausgabesignal erzeugt wird.

9. Verfahren zum Betreiben einer Vorrichtung (10) gemäß einem der vorhergehenden Ansprüche,
**dadurch gekennzeichnet, dass**
in einer Anlernphase und/oder Initialisierungsphase zumindest ein Probekörper (P) aus einem Probematerial innerhalb des Schutzgehäuses angeordnet wird und zumindest eine elektrische Eigenschaft des Probekörpers (P) über einen vorgegebenen Zeitraum unter gegebenen Umgebungseinflüssen erfasst wird, wobei das Probematerial in Korrelation gesetzt wird zu anderen im Schutzgehäuse (100) verbauten Materialien und eine Veränderung der elektrischen Eigenschaft des Probekörpers (P) und den damit korrelierten anderen Materialen bei der Ermittlung der Intensität des korrosiven Umgebungsklimas berücksichtigt wird.

## Claims

1. Device (10) for detecting a corrosive ambient atmosphere in a protective housing (100), in particular a switch cabinet (101), comprising
- at least two internal sensors (20) which are designed to measure the changes over time in different ambient parameters (T, H, G), in particular an air temperature and/or a component temperature (T1, T2, T3), an air humidity (H) and/or a gas concentration (G), inside the protective housing (100), and to generate a sensor signal (S) reflecting the respective change over time in the respective ambient parameter (T, H, G);
- an evaluation device (30) which is configured to receive the sensor signals (S) reflecting the respective changes over time in the different ambient parameters (T, H, G) and to evaluate them in correlation with one another in order to determine an intensity of the corrosive ambient atmosphere on the basis of deviations of the sensor signals (S), or of a parameter correlated therewith, from a reference value or reference value range, taking account of a time duration and/or a frequency of the deviations; and
- an output device (40) which is configured to generate an output signal reflecting the determined intensity of the corrosive ambient atmosphere;
**characterized in that**
the evaluation device (30) comprises a trained model, in particular a deep learning model, which was trained in a learning phase to classify the intensity of the corrosive ambient atmosphere on the basis of the changes over time in the different ambient parameters (T, H, G).

2. Device (10) according to one of the preceding claims,
**characterized in that**
the evaluation device (30) is designed to determine the intensity of the corrosive ambient atmosphere, taking account of a corrosion behaviour of a corroding testpiece (P) empirically determined in advance, particularly in an initialization phase and/or in the learning phase.

3. Device (10) according to one of the preceding claims,
**characterized in that**
at least one of the internal sensors (20) is configured as an air humidity sensor (22), and at least one further of the internal sensors (20) is configured as a temperature sensor (21), and the evaluation device (30) is designed to determine the intensity of the corrosive ambient atmosphere taking into account a dew point calculated on the basis of the sensor signals (S) of the air humidity sensor (22) and of the temperature sensor (22).

4. Device (10) according to Claim 3,
**characterized in that**
the temperature sensor (21) is configured to measure the temperature (T3) of a cold spot inside the protective housing (100), wherein the temperature sensor (21) defines an essentially isolated measuring point at the cold spot or is designed as a surface temperature sensor.

5. Device (10) according to Claim 3 or 4,
**characterized in that**
the temperature sensor (21) is designed as an optical sensor, in particular as an IR sensor.

6. Device (10) according to one of the preceding claims,
**characterized in that**
at least one of the internal sensors (20) is configured to measure a gas concentration (G) of a corrosive gas, in particular a chloride concentration and/or a hydrogen sulfide concentration, inside the protective housing (100).

7. Device (10) according to one of the preceding claims,
**characterized in that**
the evaluation device (30) is designed to receive local climate data (W) and/or geographical location data (L) which contain information dependent on the geographical location of the protective housing (100), and to take said data into account in determining the intensity of the corrosive ambient atmosphere.

8. Method for operating a device (10) according to one of the preceding claims,
**characterized in that**
- the changes over time in at least two different ambient parameters (T, H, G), in particular the air temperature and/or the component temperature (T1, T2, T3), the air humidity (H) and/or the gas concentration (G), are measured by means of internal sensors (20) arranged inside the protective housing, and a sensor signal (S) reflecting the respective change over time in the respective ambient parameter (T, H, G) is generated;
- the sensor signals (S) reflecting the respective changes over time in the different ambient parameters (T, H, G) are evaluated in correlation with one another; and
- the intensity of the corrosive ambient atmosphere is determined on the basis of deviations of the sensor signals (S), or of a parameter correlated therewith, from a reference value or reference value range, taking account of a time duration and a frequency of the deviations; and
- an output signal reflecting the determined intensity of the corrosive ambient atmosphere is generated.

9. Method for operating a device (10) according to one of the preceding claims,
**characterized in that**
at least one testpiece (P) made from a test material is arranged inside the protective housing in a learning phase and/or initialization phase, and at least one electrical characteristic of the testpiece (P) is measured under given ambient conditions over a predefined time period, wherein the test material is correlated with other materials installed in the protective housing (100) and a change in the electrical characteristic of the testpiece (P) and the other materials correlated therewith is taken into account in determining the intensity of the corrosive ambient atmosphere.

## Revendications

1. Arrangement (10) de reconnaissance d'un climat ambiant corrosif dans un boîtier de protection (100), en particulier une armoire électrique (101), comprenant
- au moins deux capteurs internes (20), qui sont configurés pour détecter les évolutions dans le temps de différents paramètres environnementaux (T, H, G), en particulier une température de l'air et/ou de composant (T1, T2, T3), une humidité de l'air (H) et/ou une concentration de gaz (G), à l'intérieur du boîtier de protection (100) et pour générer un signal de capteur (S) reflétant l'évolution dans le temps respective du paramètre environnemental (T, H, G) respectif ;
- un dispositif d'évaluation (30), qui est conçu pour recevoir les signaux de capteur (S) reflétant les évolutions dans le temps respectives des différents paramètres environnementaux (T, H, G) et pour les évaluer en corrélation les uns avec les autres, afin de déterminer une intensité du climat ambiant corrosif sur la base d'écarts des signaux de capteur (S) ou d'une grandeur corrélée avec ceux-ci par rapport à une valeur de consigne ou à une plage de valeurs de consigne en tenant compte d'une durée et/ou d'une fréquence des écarts ; et
- un dispositif de sortie (40), qui est conçu pour générer un signal de sortie reflétant l'intensité déterminée du climat ambiant corrosif ;
**caractérisé en ce que**
le dispositif d'évaluation (30) comprend un modèle entraîné, en particulier un modèle d'apprentissage profond, qui a été entraîné dans une phase d'apprentissage pour classifier l'intensité du climat ambiant corrosif à l'aide des évolutions dans le temps des différents paramètres environnementaux (T, H, G).

2. Arrangement (10) selon l'une des revendications précédentes,
**caractérisé en ce que**
le dispositif d'évaluation (30) est configuré pour déterminer l'intensité du climat ambiant corrosif en tenant compte d'un comportement à la corrosion d'une éprouvette (P) corrosive déterminé empiriquement au préalable, en particulier dans une phase d'initialisation et/ou dans une phase d'apprentissage.

3. Arrangement (10) selon l'une des revendications précédentes,
**caractérisé en ce que**
au moins l'un des capteurs (20) internes est conçu en tant que capteur d'humidité de l'air (22) et au moins un autre des capteurs internes (20) est conçu en tant que capteur de température (21), et le dispositif d'évaluation (30) est configuré pour déterminer l'intensité du climat ambiant corrosif en tenant compte d'un point de rosée calculé à l'aide des signaux de capteur (S) du capteur d'humidité de l'air (22) et du capteur de température (22).

4. Arrangement (10) selon la revendication 3,
**caractérisé en ce que**
le capteur de température (21) est conçu pour détecter la température (T3) d'un point froid à l'intérieur du boîtier de protection (100), le capteur de température (21) définissant un point de mesure sensiblement isolé au niveau du point froid ou étant réalisé sous forme de capteur de surface.

5. Arrangement (10) selon la revendication 3 ou 4,
**caractérisé en ce que**
le capteur de température (21) est réalisé sous la forme d'un capteur optique, notamment d'un capteur infrarouge.

6. Arrangement (10) selon l'une des revendications précédentes,
**caractérisé en ce que**
au moins l'un des capteurs internes (20) est conçu pour détecter une concentration de gaz (G) d'un gaz corrosif, en particulier une concentration de chlorure et/ou de sulfure d'hydrogène, à l'intérieur du boîtier de protection (100).

7. Arrangement (10) selon l'une des revendications précédentes,
**caractérisé en ce que**
le dispositif d'évaluation (30) est configuré pour recevoir des données climatiques locales (W) et/ou des données de localisation géographique (L), qui contiennent des informations dépendant de la localisation géographique du boîtier de protection (100), et pour en tenir compte lors de la détermination de l'intensité du climat ambiant corrosif.

8. Procédé pour faire fonctionner un arrangement (10) selon l'une des revendications précédentes,
**caractérisé en ce que**
- les évolutions dans le temps d'au moins deux paramètres environnementaux (T, H, G) différents, en particulier la température de l'air et/ou de composants (T1, T2, T3), l'humidité de l'air (H) et/ou la concentration de gaz (G), sont détectés au moyen de capteurs (20) internes disposés à l'intérieur du boîtier de protection, et un signal de capteur (S) reflétant l'évolution dans le temps respective du paramètre environnemental (T, H, G) respectif est généré ;
- les signaux de capteur (S) reflétant les évolutions dans le temps respectives des différents paramètres environnementaux (T, H, G) sont mis en corrélation les uns avec les autres pour évaluation ; et
- l'intensité du climat ambiant corrosif est déterminée à l'aide des écarts des signaux de capteur (S) ou d'une grandeur corrélée avec ceux-ci par rapport à une valeur de consigne ou à une plage de valeurs de consigne en tenant compte d'une durée et d'une fréquence des écarts ; et
- un signal de sortie reflétant l'intensité déterminée du climat ambiant corrosif est généré.

9. Procédé pour faire fonctionner un arrangement (10) selon l'une des revendications précédentes,
**caractérisé en ce que**
au cours d'une phase d'apprentissage et/ou d'une phase d'initialisation, au moins une éprouvette (P) constituée d'un matériau d'essai est disposée à l'intérieur du boîtier de protection et au moins une propriété électrique de l'éprouvette (P) est détectée pendant une période prédéfinie sous des influences environnementales prédéterminées, le matériau de l'éprouvette étant mis en corrélation avec d'autres matériaux installés dans le boîtier de protection (100) et un changement de la propriété électrique de l'éprouvette (P) et des autres matériaux corrélés avec celle-ci étant pris en compte lors de la détermination de l'intensité du climat ambiant corrosif.
